# EUROPEAN PATENT APPLICATION

(11) **EP 4 840 937 A1**
(43) Date of publication of application: **18.11.2026**
(21) Application number: 25738787.8
(22) Date of filing: 08.01.2025
(51) Int. Cl.: C12M 3/00, B32B 27/00, C12M 1/00, C12N 5/071, C12N 5/0793

(54) **CELL CULTURING VESSEL, METHOD FOR PRODUCING SAME, AND METHOD FOR CULTURING CELLS**

(30) Priority: 11.01.2024 JP 2024002531; 30.09.2024 JP 2024171160
(71) Applicant: Mitsui Chemicals, Inc., Tokyo 104-0028 (JP)
(72) Inventor: MURASE, Hirohiko, Tokyo 104-0028 (JP); ESASHIKA, Katsuhiro, Tokyo 104-0028 (JP); OKAZAKI, Takuya, Tokyo 104-0028 (JP); SAKAJIRI, Aya, Tokyo 104-0028 (JP); ANADA, Takahisa, Fukuoka 819-0395 (JP); TANAKA, Masaru, Fukuoka 819-0395 (JP)
(74) Representative: J A Kemp LLP
(86) International application number: PCT/JP2025/000396
(87) International publication number: WO 2025/150519

(57) **Abstract**

This cell culturing vessel has a base material having an oxygen permeation part, and a coating layer disposed on a surface of the base material on a side where cells are to be disposed. The oxygen permeation part is made of a resin and has an oxygen permeability coefficient of 200-2400 cm³×mm/(m²×24h×atm). The bubble contact angle of the coating layer in water is 30-100°.

## Description

### Technical Field

The present invention relates to a cell culture vessel, a method for manufacturing the cell culture vessel, and a method for culturing cells.

### Background Art

A method of increasing the culture efficiency of cells by using an oxygen-permeable cell culture vessel as a cell culture vessel for proliferating, growing, storing, and transporting cells has been studied. For example, a cell culture vessel formed by attaching an oxygen-permeable resin sheet or film to the top or bottom surface of a cylindrical or dishshaped base material is known (PTL 1 and the like).

In addition, in a cell culture vessel, a culture substrate such as laminin may be coated on a surface to which cells are to be attached (PTL 2 and the like).

### Citation List

### Patent Literature

PTL 1
   Japanese Patent Application Laid-Open No. 2016-111975
PTL 2
   Japanese Unexamined Patent Application Publication (Translation of PCT Application) No. 2023-520717

### Summary of Invention

### Technical Problem

According to the findings of the present inventors, a cell culture vessel with high oxygen permeability as described in PTL 1 may have low cell adhesiveness. As described in PTL 2, it is expected that cell adhesiveness is enhanced by coating a culture substrate, but in a cell culture vessel with high oxygen permeability, coating the culture substrate did not increase cell adhesion as much as expected.

The present invention has been made in view of the above-described problems of the related art. An object of the present invention is to provide a cell culture vessel having oxygen permeability and enhancing cell adhesiveness after a culture substrate is coated, a method for manufacturing the cell culture vessel, and a method for culturing cells using the cell culture vessel.

### Solution to Problem

One aspect of the present invention for achieving the above object relates to the cell culture vessels of [1] to [16] below.
[1] A cell culture vessel including:
   a base material including an oxygen permeation section; and
   a coating layer disposed on a surface of the base material, the surface being on a side on which cells are to be disposed, wherein
   the oxygen permeation section is made of resin and has an oxygen permeability coefficient of 200 cm³ × mm/(m² × 24 h × atm) or more and 2400 cm³ × mm/(m² × 24 h × atm) or less, and
   the coating layer has an air-bubble contact angle in water of 30° or more and 100° or less.
[2] The cell culture vessel according to [1], in which a difference between the air-bubble contact angle in water of the coating layer and an air-bubble contact angle in water of the oxygen permeation section is 35° or more and 75° or less.
[3] The cell culture vessel according to [1], in which the coating layer has a water contact angle of 40° or more and 90° or less.
[4] The cell culture vessel according to [1], in which the coating layer contains a polyalkoxyalkyl (meth)acrylate and/or a polyhydroxyalkyl (meth)acrylate.
[5] The cell culture vessel according to [4], in which the polyalkoxyalkyl (meth)acrylate contains at least one compound selected from the group consisting of polymethoxymethyl acrylate, poly(2-methoxyethyl acrylate), polymethoxypropyl acrylate, polymethoxybutyl acrylate, polyethoxymethyl acrylate, polyethoxyethyl acrylate, polyethoxypropyl acrylate, polyethoxybutyl acrylate, polypropoxymethyl acrylate, polypropoxyethyl acrylate, polypropoxypropyl acrylate, polypropoxybutyl acrylate, polybutoxymethyl acrylate, polybutoxyethyl acrylate, polybutoxypropyl acrylate, polybutoxybutyl acrylate, polymethoxymethyl methacrylate, polymethoxyethyl methacrylate, polymethoxypropyl methacrylate, polymethoxybutyl methacrylate, polyethoxymethyl methacrylate, polyethoxyethyl methacrylate, polyethoxypropyl methacrylate, polyethoxybutyl methacrylate, polypropoxymethyl methacrylate, polypropoxyethyl methacrylate, polypropoxypropyl methacrylate, polypropoxybutyl methacrylate, polybutoxymethyl methacrylate, polybutoxyethyl methacrylate, polybutoxypropyl methacrylate, and polybutoxybutyl methacrylate.
[6] The cell culture vessel according to [4] or [5], in which the polyalkoxyalkyl (meth)acrylate and/or the polyhydroxyalkyl (meth)acrylate has a number-average molecular weight Mn of 10,000 or more and 500,000 or less.
[7] The cell culture vessel according to any one of [1] to [6], in which the coating layer contains gelatin.
[8] The cell culture vessel according to any one of [1] to [7], in which the coating layer has a coating weight of 0.1 µg/cm² or more and 100 µg/cm² or less.
[9] The cell culture vessel according to any one of [1] to [8], the base material contains at least one resin selected from the group consisting of polystyrene, polyethylene, polypropylene, 4-methyl-1-pentene-based (co)polymers, and cyclic olefin-based (co)polymers.
[10] The cell culture vessel according to any one of [1] to [9], in which the oxygen permeation section has an air-bubble contact angle in water of 100° or more.
[11] The cell culture vessel according to any one of [1] to [10], in which the oxygen permeation section contains at least one resin selected from the group consisting of polyethylene, polypropylene, polyethylene terephthalate, and 4-methyl-1-pentene-based (co)polymers.
[12] The cell culture vessel according to any one of [1] to [11], in which the base material includes a frame and a resin sheet, the frame including a through-hole, the resin sheet being disposed on an upper surface or a bottom surface of the through-hole.
[13] The cell culture vessel according to [12], in which the oxygen permeation section is formed on the resin sheet.
[14] The cell culture vessel according to [12] or [13], in which the resin sheet has a thickness of 10 µm or more and 500 µm or less.
[15] The cell culture vessel according to any one of [12] to [14], in which the resin sheet is formed of a thermoplastic resin of a type different from that of the base material.
[16] The cell culture vessel according to any one of [1] to [15], in which a sterility assurance level (SAL) of a medical device, as measured in accordance with BS EN 556-1:2001, is 10⁻² or less.
   One aspect of the present invention for achieving the above object relates to the methods for manufacturing a cell culture vessel of [17] to [20] below.
[17] A method for manufacturing a cell culture vessel, the method including:
   preparing a base material including an oxygen permeation section; and
   disposing a coating layer by applying a coating material to a surface of the base material and drying the applied coating material, the surface being on a side on which cells are to be cultured,
   in which the oxygen permeation section is made of a resin and has an oxygen permeability coefficient of 200 cm³ × mm/(m² × 24 h × atm) or more and 2,400 cm³ × mm/(m² × 24 h × atm) or less, and
   the disposed coating layer has an air-bubble contact angle in water of 30° or more and 100° or less.
[18] The method according to [17], further including:
   packaging the cell culture vessel after the coating layer is disposed; and
   subjecting the packaged cell culture vessel to sterilization treatment.
[19] The method according to [17] or [18], in which the coating layer contains a polyalkoxyalkyl (meth)acrylate and/or a polyhydroxyalkyl (meth)acrylate.
[20] The method according to any one of [17] to [19], in which the coating material contains gelatin and a solvent.
   One aspect of the present invention for achieving the above object relates to the method for culturing cells of [21] below.
[21] A method for culturing cells, the method including:
   preparing the cell culture vessel according to any one of claims 1 to 16;
   forming a culture substrate layer on a surface of the coating layer, the culture substrate layer containing a natural polymer selected from the group consisting of collagen, laminin, laminin fragments, fibronectin, vitronectin, elastin, tenascin, entactin, fibrillin, and proteoglycans; and
   culturing the cell in the cell culture vessel.

### Advantageous Effects of Invention

The present invention provides a cell culture vessel having oxygen permeability and enhancing cell adhesiveness after a culture substrate is coated, a method for manufacturing the cell culture vessel, and a method for culturing cells using the cell culture vessel.

### Brief Description of Drawings

[FIG. 1] FIG. 1 is a perspective view of a cell culture vessel;
[FIG. 2] FIG. 2 is a cross-sectional view of a part of the cell culture vessel;
[FIGS. 3] FIG. 3A is a bottom perspective view of another cell culture vessel, and FIG. 3B is a top perspective view of the cell culture vessel;
[FIG. 4] FIG. 4 is a cross-sectional view of a part of the cell culture vessel;
[FIGS. 5] FIGS. 5A to 5C are microscopic photographs obtained for evaluating cell adhesiveness in Experiment 1 of the Examples, FIG. 5A is a microscopic photograph of a cell culture vessel with an evaluation of ⊚, FIG. 5B is a microscopic photograph of a cell culture vessel with an evaluation of ○, and FIG. 5C is a microscopic photograph of a cell culture vessel with an evaluation of ×; and
[FIGS. 6] FIGS. 6A to 6C are microscopic photographs obtained for evaluating cell adhesiveness in Experiment 2 of Examples, FIG. 3A is a microscopic photograph of a cell culture vessel with an evaluation of ○, FIG. 3B is a microscopic photograph of a cell culture vessel with an evaluation of △, and FIG. 3C is a microscopic photograph of a cell culture vessel with an evaluation of ×.

### Description of Embodiments

### 1. Cell culture vessel

Embodiment 1 of the present invention relates to a cell culture vessel. The cell culture vessel can be used for culturing cells or the like. In the present specification, culturing cells means proliferating and growing the cells or maintaining the cells in a state of being live cells.

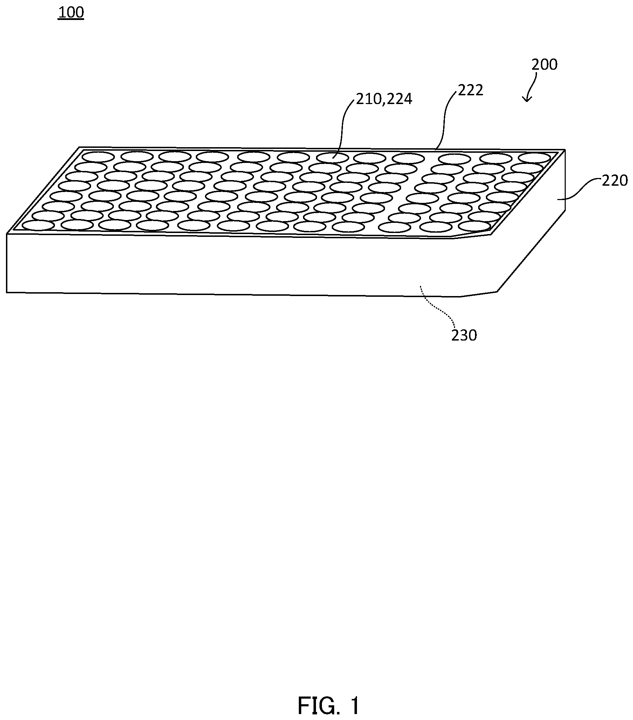

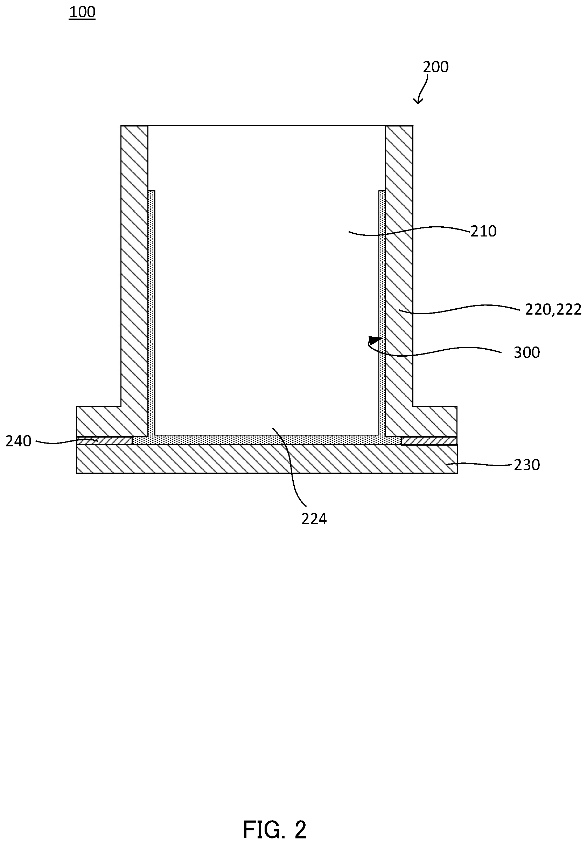

FIG. 1 is a perspective view of cell culture vessel 100, which is a cell culture vessel for culturing cells. FIG. 2 is a cross-sectional view of a part of cell culture vessel 100.

Cell culture vessel 100 is used, for example, for culturing cells derived from humans. Cell culture vessel 100 includes a plurality of accommodating sections 210 (see FIG. 2) for culturing cells. Cell culture vessel 100 accommodates an agent (culture solution) and cells to be cultured (hereinafter, referred to as "target cells") in accommodating section 210. In a state where the agent (culture solution) and the cells (target cells) are accommodated, cell culture vessel 100 is accommodated in a culture space of a culture device (for example, an incubator). It should be noted that cell culture vessel 100 does not need to be used in a state where the vessel is accommodated in a culture device. Cell culture vessel 100 may be used in various situations depending on the target cells.

Cell culture vessel 100 or at least one of resin films 112 and 114 has a sterility assurance level (SAL) of a medical device, as measured in accordance with BS EN 556-1:2001, of preferably 10⁻² or less, more preferably 10⁻³ or less, and still more preferably 10^{- 6} or less. Here, SAL represents a degree of sterility of an article to be sterilized after a sterilization process, and is expressed as a probability of microorganisms expected to be present per unit number of the articles to be sterilized after sterilization. SAL is expressed as 10⁻ⁿ, meaning that the number of viable bacteria per article to be sterilized is 10⁻ⁿ or less. Here, n is preferably 2 or more, more preferably 3 or more, and still more preferably 6 or more.

Cell culture vessel 100 includes base material 200 and coating layer 300. Base material 200 includes an oxygen permeation section, and coating layer 300 is disposed on a surface (inner surface of accommodating section 210) of base material 200-the surface on the side where cells are to be disposed.

### 1-1. Base material 200

Base material 200 includes frame 220 and resin sheet 230.

Frame 220 includes frame section 222 and a plurality of wells 224. Frame 220 is, for example, an integrally molded product made of a thermoplastic resin and formed by injection molding. Examples of the thermoplastic resin constituting frame 220 include polystyrene and polyolefins. Examples of the polyolefin include polyethylene, polypropylene, 4-methyl-1-pentene-based (co)polymers, and cyclic olefin-based (co)polymers. Among these, from the viewpoint of enhancing the gas permeability of frame 220, a 4-methyl-1-pentene-based polymer is preferable. In addition, from the viewpoint of enhancing the heat resistance of frame 220, a 4-methyl-1-pentene-based polymer, polystyrene, polypropylene, or a cyclic olefin-based polymer is preferable. As the 4-methyl-1-pentene-based polymer, the 4-methyl-1-pentene-based polymer described as the material of the adhesion layer of the sheet for a cell culture vessel can be used in the same manner, but a polymer different from the material of the adhesion layer may be used.

Frame section 222 is constituted by a rectangular frame-shaped member. Well 224 is provided in a space surrounded by frame section 222. Well 224 is provided integrally with frame section 222.

Well 224 is a cylindrical through-hole that opens upward. In the present embodiment, the shape of the opening portion of well 224 (in other words, the outline thereof in plan view) is circular. Wells 224 are provided side by side in the left-right direction and the front-back direction. The number of wells 224 may be, for example, 6, 24, 96, or 384. Naturally, the number of wells 224 may be other than 6, 24, 96, and 384.

Adjacent wells 224 are connected to each other by connection section 326. Further, well 224 at the left end, well 224 at the right end, well 224 at the front end, and well 224 at the rear end are connected to the inner peripheral surface of frame section 222 via a connection section (not illustrated).

Resin sheet 230 is attached to the lower surface of frame 220 via pressure-sensitive adhesive layer 240. As a result, resin sheet 230 closes the upper surface or the bottom surface of cylindrical section 310 (through-hole) to constitute the upper surface or the bottom surface of container 1. In the present embodiment, resin sheet 230 is formed of a material having high oxygen permeability. As a result, resin sheet 230 also functions as an oxygen permeation section that supplies oxygen to cells cultured inside accommodating section 210.

Resin sheet 230 is a sheet of a transparent resin having high oxygen permeability. Examples of such a transparent resin include polyethylene, polypropylene, polyethylene terephthalate, and 4-methyl-1-pentene-based (co)polymers.

It is preferable that the resin serving as the material of resin sheet 230 is a thermoplastic resin different from the thermoplastic resin serving as the material of frame 220. For example, the thermoplastic resin serving as the material of frame 220 may be polystyrene or a cyclic olefin-based (co)polymer, and the resin serving as the material of resin sheet 230 may be a 4-methyl-1-pentene-based (co)polymer. Alternatively, the thermoplastic resin serving as the material of frame 220 may be a 4-methyl-1-pentene-based (co)polymer, and the resin serving as the material of resin sheet 230 may be a 4-methyl-1-pentene-based (co)polymer with a different comonomer type or ratio. By using different thermoplastic resins for these materials, different characteristics can be imparted to frame 220 and resin sheet 230. For example, by imparting rigidity to frame 220 and imparting oxygen permeability to resin sheet 230, cell culture vessel 100 that has both rigidity and oxygen permeability can be obtained.

From the viewpoint of creating aerobic conditions inside accommodating section 210 to increase the metabolic activity of cells therein, it is preferable that resin sheet 230 has an oxygen permeability coefficient of 200 cm³ × mm/(m² × 24 h × atm) or more and 2400 cm³ × mm/(m² × 24 h × atm) or less. Further, from the same viewpoint, it is preferable that resin sheet 230 has an oxygen transmission rate of 4500 cm³/(m² × 24 h × atm) or more and 120000 cm³/(m² × 24 h × atm) or less. From the viewpoint of effectively increasing oxygen permeability, it is preferable that resin sheet 230 is a sheet-shaped member including a polymer having a constitutional unit derived from 4-methyl-1-pentene.

The oxygen permeability coefficient is a value obtained by measuring the oxygen transmission rate [cm³/(m² × 24 h × atm)] at a temperature of 23°C and a relative humidity of 0% by a differential pressure gas permeability measurement method and dividing the oxygen transmission rate by the thickness (mm) of resin sheet 230. The device used for measurement is not particularly limited as long as it uses a differential pressure gas permeability measurement method, and for example, BT-3MT-C3 manufactured by Toyo Seiki Seisaku-sho, Ltd. can be used. A measurement sample is created by cutting out a test piece of 90 × 90 mm from resin sheet 230, and the measurement section diameter is set to 70 mm (permeation area is 38.46 cm²). When it is expected that the oxygen transmission rate is high, it is more preferable to apply an aluminum mask to the measurement sample in advance and set the actual permeation area to 5.0 cm².

From the viewpoint of increasing the observation efficiency of cells, it is preferable that resin sheet 230 is transparent. Specifically, the total light transmittance measured in accordance with JIS K 7361-1 is preferably 50% or more and 100% or less, and more preferably 70% or more and 100% or less.

Resin sheet 230 preferably has an air-bubble contact angle in water before the sterilization treatment of 90° or more and 120° or less, more preferably 95° or more and 115° or less, and even more preferably 100° or more and 110° or less. In addition, resin sheet 230 preferably has an air-bubble contact angle in water after the sterilization treatment of 90° or more, more preferably 95° or more and 115° or less, and even more preferably 100° or more and 110° or less.

The thickness of resin sheet 230 is not particularly limited, but is preferably 10 µm or more and 500 µm or less, more preferably 20 µm or more and 300 µm or less, and still more preferably 20 µm or more and 200 µm or less. As the thickness increases, the strength of resin sheet 230 can be increased. As the thickness decreases, the oxygen permeability of resin sheet 230 can be increased.

Pressure-sensitive adhesive layer 240 attaches resin sheet 230 to the lower surface of a bottom plate section in accommodating section 210. A known adhesive such as an acrylic, silicone, urethane, or rubber-based adhesive can be used for pressure-sensitive adhesive layer 240. Among these, an acrylic and silicone-based adhesives are preferable.

### 1-2. Coating Layer 300

Coating layer 300 is a coating made of a hydrophilic material disposed inside accommodating section 210. Coating layer 300 may be disposed to be in contact with the oxygen permeation section, may be disposed to be in contact with a portion that is not the oxygen permeation section, or may be disposed to be in contact with both of the oxygen permeation section and the other portion. For example, in the present embodiment, coating layer 300 may be disposed to be in contact with resin sheet 230 (oxygen permeation section) and with frame 220 (portion that is not the oxygen permeation section).

Coating layer 300 may be a coating for imparting hydrophilicity to the inner surface of accommodating section 210 to enhance cell adhesiveness. From this viewpoint, coating layer 300 preferably contains a water absorbent resin or gelatin. Examples of the water absorbent resin include poly(vinyl alcohol) (PVA), poly(ethylene glycol) (PEG), PEG-acrylate, poly(vinylpyrrolidone) (PVP), polyethyleneimine (PEI), poly(L-lactide) (PLLA), poly(D-lactide) (PDLA), poly(L-lactide-co-D,L-lactide) (PLDLLA), poly(glycolic acid) (PGA), poly(lactic acid-co-glycolic acid) (PL-co-GA), poly(methyl methacrylate) (PMMA), polyalkoxyalkyl (meth)acrylates, polyhydroxyalkyl (meth)acrylates, and poly(2-methacryloyloxyethyl phosphorylcholine) (PMPC). Among these, from the viewpoint of further enhancing cell adhesiveness, polyalkoxyalkyl (meth)acrylates, polyhydroxyalkyl (meth)acrylates, and gelatin are preferable, and polyalkoxyalkyl (meth)acrylates and polyhydroxyalkyl (meth)acrylates are more preferable.

Examples of the polyalkoxyalkyl (meth)acrylates include polymethoxymethyl acrylate, poly(2-methoxyethyl acrylate) (PMEA), polymethoxypropyl acrylate, polymethoxybutyl acrylate, polyethoxymethyl acrylate, polyethoxyethyl acrylate, polyethoxypropyl acrylate, polyethoxybutyl acrylate, polypropoxymethyl acrylate, polypropoxyethyl acrylate, polypropoxypropyl acrylate, polypropoxybutyl acrylate, polybutoxymethyl acrylate, polybutoxyethyl acrylate, polybutoxypropyl acrylate, polybutoxybutyl acrylate, polymethoxymethyl methacrylate, polymethoxyethyl methacrylate, polymethoxypropyl methacrylate, polymethoxybutyl methacrylate, polyethoxymethyl methacrylate, polyethoxyethyl methacrylate, polyethoxypropyl methacrylate, polyethoxybutyl methacrylate, polypropoxymethyl methacrylate, polypropoxyethyl methacrylate, polypropoxypropyl methacrylate, polypropoxybutyl methacrylate, polybutoxymethyl methacrylate, polybutoxyethyl methacrylate, polybutoxypropyl methacrylate, and polybutoxybutyl methacrylate. Among these, poly(2-methoxyethyl acrylate) (PMEA) is preferable.

Examples of the polyhydroxyalkyl (meth)acrylates include polyhydroxyethyl methacrylate (PHEMA), polyhydroxypropyl methacrylate, and polyhydroxybutyl methacrylate. Among these, polyhydroxyethyl methacrylate (PHEMA) is preferable.

Among these, from the viewpoint of further enhancing cell adhesiveness, polymethoxyethyl acrylate, poly(hydroxyethyl methacrylate), and gelatin are preferable, and polymethoxyethyl acrylate is more preferable.

In addition, these may be combined, and for example, a polyalkoxyalkyl (meth)acrylate and a polyhydroxyalkyl (meth)acrylate may be used in combination. When combined, it is preferable to combine polymethoxyethyl acrylate and poly(hydroxyethyl methacrylate). The ratio (mass ratio) when these are combined is preferably 1:99 or more and 99:1 or less (polymethoxyethyl acrylate:poly(hydroxyethyl methacrylate)), more preferably 3:97 or more and 97:3 or less, and still more preferably 5:95 or more and 95:5 or less, .

The polyalkoxyalkyl (meth)acrylate and/or polyhydroxyalkyl (meth)acrylate preferably has a number-average molecular weight (Mn) of 10,000 to 500,000, more preferably 20,000 to 400,000, still more preferably 30,000 to 350,000, and particularly preferably 30,000 to 250,000. The larger the number-average molecular weight (Mn), the better the uniformity of the thickness of the coating layer becomes, and the smaller the number-average molecular weight (Mn), the better the dispersibility of the polyalkoxyalkyl (meth)acrylate and/or polyhydroxyalkyl (meth)acrylate becomes in the solvent.

Examples of gelatin include beef bone-derived gelatin, beef skin-derived gelatin, pork skin-derived gelatin, donkey skin-derived gelatin, chicken skin-derived gelatin, and fish-derived gelatin. Pork skin-derived gelatin is preferable from the viewpoint of cell adhesiveness. These may be used alone or in combination of two or more.

Coating layer 300 has an air-bubble contact angle in water of 30° or more and 100° or less, preferably 30° or more and 70° or less, and more preferably 30° or more and 55° or less. The larger the air-bubble contact angle in water of coating layer 300, the more appropriate the moisture content of coating layer 300 becomes, and the less likely it is for the cell contact property to decrease. The smaller the air-bubble contact angle in water of coating layer 300, the more appropriately the hydrophilicity can be imparted to the inner surface of the accommodating section 210, and the cell adhesiveness can be enhanced. Further, the culture substrate is applied to the surface of coating layer 300 by an aqueous coating liquid. Therefore, the smaller the air-bubble contact angle in water of coating layer 300, the easier it is to adhere the culture substrate to coating layer 300, and the more it is possible to suppress the decrease in cell adhesiveness due to the peeling of the culture substrate. The air-bubble contact angle in water can be adjusted by the material of coating layer 300, the conditions of the pretreatment applied to cell culture vessel 100 before disposing coating layer 300, and the like.

Coating layer 300 has a water contact angle of preferably 40° or more and 90° or less, preferably 45° or more and 90° or less, and more preferably 45° or more and 60° or less. The culture substrate is applied to the surface of coating layer 300 by an aqueous coating liquid. Therefore, the smaller the water contact angle of coating layer 300, the easier it is to adhere the culture substrate to coating layer 300, and the more it is possible to suppress the decrease in cell adhesiveness due to the peeling of the culture substrate. The water contact angle can be adjusted by the material of coating layer 300, the conditions of the pretreatment applied to cell culture vessel 100 before disposing coating layer 300, and the like.

Further, the difference between the air-bubble contact angle in water of coating layer 300 and the air-bubble contact angle in water of resin sheet 230 (oxygen permeation section) is preferably 35° or more and 75° or less, more preferably 40° or more and 70° or less, still more preferably 45° or more and 65° or less, and particularly preferably 45° or more and 60° or less. When the difference between these air-bubble contact angles is small, the adherence of coating layer 300 to resin sheet 230 is high, and the peeling of coating layer 300 from the accommodating section 210 is less likely to occur.

Note that the difference between the air-bubble contact angles in water of coating layer 300 and resin sheet 230 described above is a difference in air-bubble contact angle measured after cell culture vessel 100, in which coating layer 300 is formed, is subjected to sterilization treatment (for example, terminal sterilization). The sterilization treatment of cell culture vessel 100 is performed by irradiation with electron beams, gamma rays, or the like. When coating layer 300 and/or base material 200 (resin sheet 230 or the like) is deteriorated by the sterilization treatment by these methods, the adherence between these components may decrease, coating layer 300 may peel off, and the cell adhesiveness may be less likely to increase. Further, when coating layer 300 is formed of a material having a hydrophilic group such as a hydroxyl group or a carboxyl group, crosslinking may occur during the sterilization treatment, increasing the density of the hydrophilic group, which may increase the hydrophilicity of coating layer 300 excessively, leading to an increase in moisture content and a decrease in cell adhesiveness.

For such a problem, when the difference between the air-bubble contact angles of coating layer 300 and resin sheet 230 after the sterilization treatment is small, the difference in surface energy between coating layer 300 and base material 200 (resin sheet 230) is small even after the sterilization treatment, and the high adherence between these components can be maintained. Further, by making the air-bubble contact angle of coating layer 300 appropriately close to the air-bubble contact angle of hydrophobic base material 200 (resin sheet 230), it is also possible to prevent a decrease in the cell contact property due to an increase in moisture content caused by excessive hydrophilicity of the coating layer 300 due to sterilization treatment.

The method for controlling the difference in air-bubble contact angle is not limited. For example, for coating layer 300 formed of a polyalkoxyalkyl (meth)acrylate, the difference in air-bubble contact angle can be adjusted by gamma ray irradiation of the coating layer, or by plasma treatment or corona treatment after gamma ray irradiation. In addition, for coating layer 300 formed of gelatin, the difference in air-bubble contact angle can be adjusted by storing the coating layer at room temperature for about 2 to 3 weeks after formation to remove radicals remaining in the gelatin.

Further, for coating layer 300 formed of a polyhydroxyalkyl (meth)acrylate, the difference in air-bubble contact angle can be adjusted by increasing the coating amount of the polyhydroxyalkyl (meth)acrylate or by gamma ray irradiation.

It is known that a cell culture vessel is subjected to a plasma treatment or the like in order to enhance the adhesiveness of cells. However, according to the findings of the present inventors, even when a cell culture vessel is subjected to a plasma treatment as in the related art, the adhesiveness of cells is not increased in many cases, and even when the cell adhesiveness is increased, the effect is more likely to be lost in a short period of time. The reason for this is not clear, but it is considered that a material having high oxygen permeability, such as resin sheet 230, is often amorphous, and functional groups formed by a plasma treatment or the like are easily taken into the inside. Perhaps due to the above characteristics, a culture substrate is difficult to adhere to the cell culture vessel, or even when the culture substrate adheres, it is more likely to peel off over time, or the adherence of the cell culture vessel is significantly reduced after storing the cell culture vessel. Therefore, it is considered that the adhesiveness of cells is not sufficiently increased, either. On the other hand, by providing coating layer 300 with an air-bubble contact angle in water within the above range, the adherence of the culture substrate can be sufficiently increased, and the deterioration of adherence over time is also less likely to occur.

The coating weight of coating layer 300 can be calculated by the concentration (µg/mL) of the material of coating layer 300 in the coating material × the coating amount (mL) × the area of the well bottom (cm²). The coating weight of coating layer 300 is not particularly limited, but is preferably 0.1 µg/cm² or more and 100 µg/cm² or less, more preferably 0.15 µg/cm² or more and 80 µg/cm² or less, and still more preferably 0.2 µg/cm² or more and 70 µg/cm² or less. The higher the coating weight of coating layer 300, the more the adhesiveness of cells can be enhanced. The lower the coating weight of coating layer 300, the higher the oxygen permeability can be enhanced.

Cell culture vessel 100 has an oxygen permeability coefficient through coating layer 300 after coating layer 300 is disposed of 200 cm³ × mm/(m² × 24 h × atm) or more and 2400 cm³ × mm/(m² × 24 h × atm) or less, more preferably 500 cm³ × mm/(m² × 24 h × atm) or more and 2400 cm³ × mm/(m² × 24 h × atm) or less, and still more preferably 1200 cm³ × mm/(m² × 24 h × atm) or more and 2400 cm³ × mm/(m² × 24 h × atm) or less. In addition, from the same viewpoint, cell culture vessel 100 has an oxygen transmission rate through coating layer 300 of preferably 3000 cm³/(m² × 24 h × atm) or more and 40000 cm³/(m² × 24 h × atm) or less, more preferably 3300 cm³/(m² × 24 h × atm) or more and 36000 cm³/(m² × 24 h × atm) or less, and still more preferably 3600 cm³/(m² × 24 h × atm) or more and 32000 cm³/(m² × 24 h × atm) or less.

The oxygen permeability coefficient is a value obtained by measuring the oxygen transmission rate [cm³/(m² × 24 h × atm)] at a temperature of 23°C and a relative humidity of 0% by a differential pressure gas permeability measurement method and dividing the oxygen transmission rate by the thickness (mm) of resin sheet 230. The device used for measurement is not particularly limited as long as it uses a differential pressure gas permeability measurement method, and for example, BT-3MT-C3 manufactured by Toyo Seiki Seisaku-sho, Ltd. can be used. The measurement sample is preferably created by cutting out a 90 × 90 mm test piece from a resin sheet having a thickness of 50 µm, and the measurement section diameter is preferably 70 mm (permeation area of 38.46 cm²). Since the oxygen transmission rate is high, it is more preferable to apply an aluminum mask to the sample in advance and set the actual permeation area to 5.0 cm². The resin sheet may or may not have been subjected to microfabrication and/or surface modification treatment, but it is preferable that no treatment is performed.

### 1-3. Culture substrate layer

When culturing cells, it is preferable to form a culture substrate layer on the surface of coating layer 300. The cell culture vessel has satisfactory adherence to the culture substrate layer and high adhesiveness to the cells.

The culture substrate layer is preferably a layer containing a natural polymer. Examples of the natural polymer include collagen, laminin, laminin fragments, fibronectin, vitronectin, elastin, tenascin, entactin, fibrillin, and proteoglycans. Among these, laminin is preferable and laminin fragments are more preferable because of satisfactory adhesion property to the coating layer having the above-described characteristics and high cell adhesiveness.

The coating weight of the culture substrate layer is not particularly limited, but is preferably 0.1 µg/cm² or more and 100 µg/cm² or less, more preferably 0.2 µg/cm² or more and 70 µg/cm² or less, and still more preferably 0.3 µg/cm² or more and 50 µg/cm² or less. The higher the coating weight of the culture substrate layer, the more the cell adhesiveness can be enhanced. The lower the coating weight of the culture substrate layer, the more the oxygen permeability can be enhanced.

### 2. Method of producing cell culture vessel

Cell culture vessel 100 can be produced by forming coating layer 300 on base material 200 including oxygen permeation section (resin sheet 230).

Base material 200 may be prepared by a known method. For example, base material 200 described above can be produced by attaching resin sheet 230 to the bottom surface of frame 220 with pressure-sensitive adhesive layer 240 interposed therebetween.

Coating layer 300 can be formed by applying a coating material containing the material of coating layer 300 described above to the surface (inner surface of accommodating section 210) of base material 200-the surface on the side where the cells are to be cultured.

The coating material may be a treatment liquid containing the above-described material and an aqueous solvent. The type of the solvent is not particularly limited, but when the coating material contains a polyalkoxyalkyl (meth)acrylate and/or a polyhydroxyalkyl (meth)acrylate, it is preferable that the solvent contains water and a primary alcohol. By using a solvent containing a primary alcohol, the coating uniformity of the coating material can be improved. Examples of the primary alcohol include methanol, ethanol, 1-propanol, and 1-butanol. The ratio of water to the primary alcohol in the solvent is preferably 20:80 or more and 80:20 or less, and more preferably 40:60 or more and 60:40 or less in terms of volume ratio (water: primary alcohol). When the material of coating layer 300 is gelatin, the solvent may be composed of only water.

The concentration of the material of coating layer 300 in the coating material is preferably 0.0005 mg/mL or more and 0.5 mg/mL or less, more preferably 0.001 mg/mL or more and 0.3 mg/mL or less, and still more preferably 0.02 mg/mL or more and 0.3 mg/mL or less. As the concentration of coating layer 300 is increased, the effect of improving the cell adhesiveness by coating layer 300 can be enhanced. By setting the concentration of coating layer 300 to an appropriate range, it is possible to easily form coating layer 300 uniformly.

The coating weight of the coating material may be adjusted according to the coating weight of coating layer 300.

A pretreatment may be performed on the surface of base material 200 on the side where the cells are to be cultured before applying the coating material. As the pretreatment, a plasma treatment, a corona discharge, a UV ozone treatment, or the like can be performed. By these pretreatments, the air-bubble contact angle in water of coating layer 300 and the adherence to the base material can be adjusted. For example, in the case of a plasma treatment, the treatment conditions can be set to an irradiation time of 1 second or more and 15 seconds or less, an output of 10 V or more and 150 V or less, and a distance between the container bottom surface and the torch of 1 mm or more and 15 mm or less.

It is preferable to sterilize base material 200 after forming coating layer 300 by applying and drying the coating material. It is more preferable to perform the sterilization treatment after packaging the cell culture vessel (terminal sterilization). The sterilization treatment can be performed by a known method such as irradiation with electron beams or gamma rays, and may be performed by, for example, gamma ray irradiation with a dose of 12 kGy.

The air-bubble contact angles in water of resin sheet 230 and coating layer 300 after sterilization and the like can be adjusted by adjusting conditions of the sterilization treatment, the post-treatment after the sterilization treatment, and the like. For example, when coating layer 300 contains a polyalkoxyalkyl (meth)acrylate and/or a polyhydroxyalkyl (meth)acrylate, the air-bubble contact angle in water of coating layer 300 after sterilization can be adjusted by performing sterilization treatment by electron beam irradiation or performing a pretreatment before applying the coating material. In addition, when coating layer 300 contains gelatin, the air-bubble contact angle in water of coating layer 300 can be adjusted by removing radicals remaining in the gelatin by storing coating layer 300 at room temperature for about 2 to 3 weeks after the sterilization treatment.

### 3. Method for culturing cells

The above-described cell culture vessel can be used for culturing cells and the like.

When culturing cells, it is preferable to form a culture substrate layer on the surface of coating layer 300. The above-described cell culture vessel has satisfactory adherence of the culture substrate layer and high adhesiveness of cells.

The culture substrate layer is preferably a layer containing a natural polymer. Examples of the natural polymer include collagen, laminin, laminin fragments, fibronectin, vitronectin, elastin, tenascin, entactin, fibrillin, and proteoglycans.

Laminin is a cross-shaped molecule in which α, β, and γ chains are associated in a coiled-coil region, and several types exist for each chain. Therefore, five types of α chains (LAMA1 to 5), four types of β chains (LAMB1 to 4), and three types of γ chains (LAMC1 to 3) are combined and referred to, and laminin 511 means laminin α5β1γ1. Laminin exhibits a unique action. As laminin, it is preferable to use laminin 511 or laminin 521. The origin of laminin is not particularly limited, and various laminins derived from living organisms can be used. The full-length laminin or a laminin fragment can be used, and from the viewpoint of enhancing cell adhesiveness, it is preferable to use a laminin fragment.

The culture substrate layer can be formed by applying the above-described treatment liquid containing a natural polymer and water and drying the treatment liquid.

The culture of cells may be performed by accommodating an agent (culture solution) and target cells in each well 224 and accommodating the agent and the target cells in the culture space of the culture device (incubator) with conditions such as a predetermined temperature having been set.

### 4. Other Embodiments

Each of the above-described embodiments is an example of the present invention, and the present invention is not limited to each of the above-described embodiments. It goes without saying that various other embodiments are also possible within the scope of the concept of the present invention.

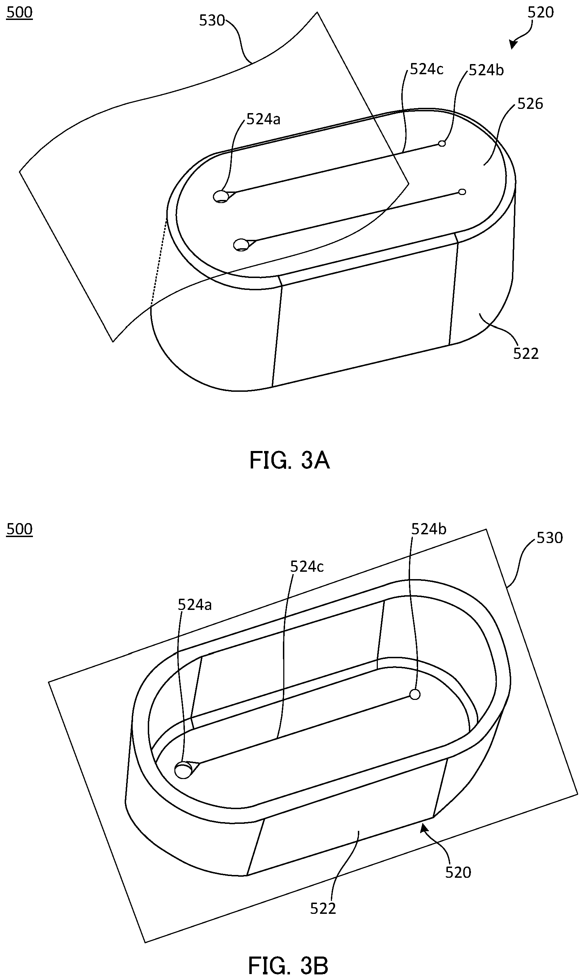

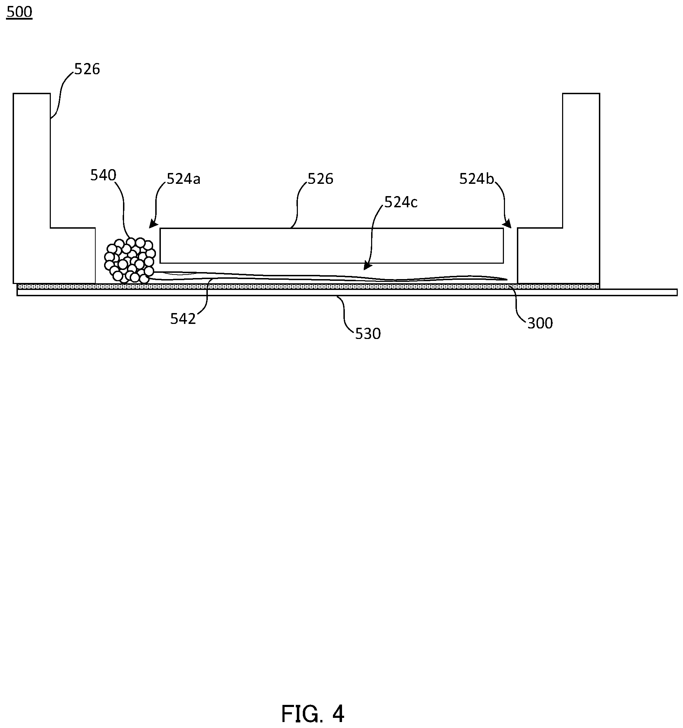

FIG. 3A is a bottom perspective view of an example of another cell culture vessel 500. FIG. 3B is a top perspective view of cell culture vessel 500. FIG. 4 is a cross-sectional view of a part of cell culture vessel 500.

Cell culture vessel 500 is used, for example, for culturing neural cells. Cell culture vessel 500 includes, for example, chamber 524a and chamber 524b in which cell body 540 of immature neural cells induced and differentiated from stem cells is to be disposed, and flow path 524c that allows chamber 524a and chamber 524b to communicate each other. Cell culture vessel 500 is accommodated in a culture space of a culture device (for example, an incubator) in the following state: an agent (culture solution) is accommodated in chamber 524a, chamber 524b, and flow path 524c, and a mass of cell bodies 540 of neural cells, which are target cells to be cultured, is accommodated in one of chamber 524a and chamber 524b.

Cell bodies 540 accommodated in the chamber each grow axon 542 along flow path 524c. At this time, axons 542 are self-organized and bundled together to extend, thereby forming neural tissue (a step of culturing cells).

Cell culture vessel 500 includes frame 520 and resin sheet 530. Resin sheet 530 is a transparent resin sheet having oxygen permeability.

Frame 520 includes frame section 522 and bottom plate portion 528. At positions of bottom plate portion 528 corresponding to chambers 524a, 524b and flow path 524c, through-holes are formed in the bottom plate portion 528 in the shapes of these chambers and flow path. Frame 520 is, for example, an integrally molded product formed by injection molding from the same synthetic resin as frame 220.

As illustrated in FIG. 3B, resin sheet 530 is disposed on the bottom surface of frame 520 (the bottom surface of chamber 524a, chamber 524b, and flow path 524c) and is pressed to be in close contact with the bottom surface of frame 520. As a result, cell culture vessel 500 including resin sheet 530 as a bottom material for chamber 524a, chamber 524b, and flow path 524c is formed.

As illustrated in FIG. 4, coating layer 300 is disposed on the surface of resin sheet 530-the surface on the side where the cells are to be disposed (the surface on the chamber 524a, chamber 524b, and flow path 524c side). Coating layer 300 has characteristics as described above.

Cell culture vessel 500 having such a configuration can also be used to culture cells by forming a culture substrate layer. The adherence of the cells is enhanced by coating layer 300 having the above-described characteristics.

For example, the above-described cell culture vessel is a cell culture vessel for proliferating or growing cells, but a container for storing or transporting cells or for observing cells may also be used as a cell culture vessel. In addition, the cells targeted by the cell culture vessel are not particularly limited, and may be differentiated somatic cells or undifferentiated stem cells. The cells may be live cells or dead cells.

### Examples

The present invention will be described in detail based on Examples, but the present invention is not limited to these Examples.

The glass transition point (Tg) of each material used below is a value obtained from the peak top of the loss elastic modulus by measuring the material using a dynamic viscoelasticity measuring device (DVA-225 manufactured by IT Keisoku Seigyo Co., Ltd.) in a tensile mode, at a temperature of -30°C to 200°C (heating rate of 3°C/min), a frequency of 1 Hz, and in an atmospheric environment.

In addition, the number-average molecular weight (Mn) of each material used below is a value measured by gel permeation chromatography (GPC). Specifically, the number-average molecular weight (Mn) of a polymer dissolved in dimethylformamide (DMF) was calibrated with a polystyrene standard under the following conditions to obtain the molecular weight.
- Column: TSKgel SuperAWM-H, 2 pieces (manufactured by Tosoh Corporation)
- Column temperature: 40°C
- Mobile phase: DMF for GPC (manufactured by FUJIFILM Wako Pure Chemical Corporation), 10 mM LiBr added
- Detector: Differential refractometer
- Flow rate: 0.6 mL/min
- Sample concentration: 1.0 mg/mL
- Injection volume: 20 µL
- Column calibration: Monodisperse polystyrene (EasiVial PS-H polystyrene (manufactured by Agilent Technologies, Inc.))
- Molecular weight calibration: Relative calibration method (polystyrene equivalent)

### 1. Production of cell culture vessel

4-Methyl-1-pentene polymer (TPX, manufactured by Mitsui Chemicals, Inc. ("TPX" is a registered trademark of Mitsui Chemicals, Inc.), molecular weight (Mw) = 428,000, molecular weight distribution (Mw/Mn) = 4.1, Tg = 40°C) was put into an extruder with a T-die equipped with a full flight type screw, the extrusion temperature was set to 270°C, the roll temperature was set to 60°C, and the roll rotation speed was adjusted to perform extrusion molding, thereby obtaining a resin sheet having a thickness of 50 µm.

The thickness of the resin sheet was obtained as the average of thicknesses measured at five points of the resin sheet using a digital micrometer (MDC-25MXT, manufactured by Mitutoyo Corporation).

The resin sheet obtained above was closely attached to the bottom surface of a 24-well container frame (frame) made of polystyrene (hereinafter, also simply referred to as "PS") having a glass transition point of 94°C via an acrylic pressure-sensitive adhesive to produce a 24-well culture plate (cell culture vessel). Thereafter, the culture plate was disposed in a chamber of a normal pressure plasma surface treatment apparatus (manufactured by Sekisui Chemical Co., Ltd.), the chamber was filled with a nitrogen gas flow, and the culture plate was subjected to a plasma treatment (irradiation time: 2 seconds, output: 10 V, distance between the container bottom surface and the torch: 2 mm).

The oxygen transmission rate and the oxygen permeability coefficient of the sterilized resin sheet were measured using a differential pressure type gas permeability measuring device (BT-3MT-C3, manufactured by Toyo Seiki Seisaku-sho, Ltd.) in an environment of a temperature of 23°C and a humidity of 0% RH. The measurement section diameter was set to 70 mm (permeation area: 38.47 cm²). Since it was expected that the oxygen permeability coefficient would be large, an aluminum mask was applied to the sample in advance, and the actual permeation area was set to 5.0 cm².

### 2. Preparation of coating material

### 2-1. Type of coating material

The following materials were prepared.

### (1) Polyalkoxyalkyl (meth)acrylate

- PMEA1: poly(2-methoxyethyl acrylate) with a number-average molecular weight Mn of 74,000 (manufactured by POLYMER SOURCE)
- PMEA2: poly(2-methoxyethyl acrylate) with a number-average molecular weight Mn of 305,000 (manufactured by POLYMER SOURCE)

### (2) Polyhydroxyalkyl (meth)acrylate

- Poly(2-hydroxyethyl methacrylate) (PHEMA) (manufactured by Sigma-Aldrich Japan)

### (3) Gelatin

- Powder gelatin (beMatrix Gelatin LS-250, manufactured by Nitta Gelatin Inc.)

### 2-2. Preparation of coating material

PHEMA (1 g) was dissolved in a mixed solution of 10 mL of ethanol and 10 mL of water for injection and stirred with a stirrer for 1 hour. Thereafter, the solution was diluted with ethanol and water (volume ratio of 1:1) and sterilized by filtration, thereby preparing a coating material. At this time, the concentration of PHEMA after dilution was changed to obtain coating materials 1-1 to 1-4.

To 50 mL of water for injection (Japanese Pharmacopoeia, manufactured by Otsuka Pharmaceutical Co., Ltd.), 50 mg of powder gelatin (beMatrix Gelatin LS-250, manufactured by Nitta Gelatin Inc.) was added and allowed to stand at room temperature for 30 minutes to swell. Thereafter, the bottle containing the swollen gelatin was heated in a hot water bath at 50°C for 1 hour to dissolve the gelatin, and further diluted with water for injection to prepare a coating material. At this time, the concentration of the gelatin after dilution was changed to obtain coating materials 1-5 to 1-7 and coating material 2-5.

To 3 mg of any one of the polyalkoxyalkyl (meth)acrylates and polyhydroxyalkyl (meth)acrylates, 0.5 mL of ethanol and 0.5 mL of water for injection were added, thereby preparing an ethanol/water solution of the polyalkoxyalkyl (meth)acrylate or polyhydroxyalkyl (meth)acrylate with a concentration of 3 mg/mL. Thereafter, the solution was diluted with ethanol and water (volume ratio of 1:1) to obtain coating materials 2-1 to 2-4.

Coating material 2-6 was prepared by mixing an 80 vol% ethanol/water solution of poly(2-methoxyethyl acrylate) (PMEA1) with a concentration of 30 µg/mL and a 20 vol% ethanol/water solution of poly(2-hydroxyethyl methacrylate) (PHEMA) with a concentration of 30 µg/mL. The ratio (mass ratio) of PMEA1 to PHEMA in the coating material 2-6 is 80:20.

Coating material 2-7 was prepared by mixing an 20 vol% ethanol/water solution of poly(2-methoxyethyl acrylate) (PMEA1) with a concentration of 30 µg/mL and a 80 vol% ethanol/water solution of poly(2-hydroxyethyl methacrylate) (PHEMA) with a concentration of 30 µg/mL. The ratio (mass ratio) of PMEA1 to PHEMA in the coating material 2-7 is 20:80.

A 0.1 M hydrochloric acid solution (for volumetric analysis, manufactured by FUJIFILM Wako Pure Chemical Corporation) was diluted 100 times with water for injection (Japanese Pharmacopoeia, manufactured by Otsuka Pharmaceutical Co., Ltd.) to prepare a 0.001 M hydrochloric acid solution. The prepared hydrochloric acid solution was sterilized by filtration. A collagen solution (Cell Matrix Type I-P, bovine tendon-derived, manufactured by Nitta Gelatin Inc.) with a concentration of 3 mg/mL was diluted with the above 0.001 M hydrochloric acid solution to prepare coating materials 2-8 and 2-9.

### 3. Disposition of Coating Layer

### 3-1. Experiment 1

In Experiment 1, when creating the cell culture vessel, the culture plate to which the resin sheet was attached was packaged in a gamma ray-resistant bag and irradiated with 10 kGy of gamma rays for sterilization treatment. Then, each of the coating materials 1-1 to 1-9 was applied to wells of the sterilized cell culture vessel with a pipette such that the application amount was 200 µL. Thereafter, the cell culture vessel was dried at room temperature overnight to obtain each of cell culture vessels 1-1 to 1-9 in which the coating layer was disposed.

### 3-2. Experiment 2

In Experiment 2, the culture plate was not sterilized before the application of a coating material, and instead, each of the cell culture vessels after the application and drying of the coating material was packaged in a gamma ray-resistant bag and irradiated with 12 kGy of gamma rays for sterilization treatment (terminal sterilization), thereby obtaining each of cell culture vessels 2-1 to 2-9 in which the coating layer was disposed. The cell culture vessel 2-5 was stored in an environment of 23°C and 50% RH for 3 weeks, and then the subsequent evaluation was performed.

In addition, a cell culture vessel in which the coating layer was not disposed was packaged in a gamma ray-resistant bag and irradiated with 12 kGy of gamma rays for sterilization treatment to obtain a cell culture vessel 2-10.

### 4. Measurement and evaluation

### 4-1. Evaluation of Experiment 1

### 4-1-1. Air-bubble contact angle in water

The air-bubble contact angle in water of the obtained coating layer surface (in the case of cell culture vessel 1-10, the surface of the resin sheet on the side where cells are to be disposed) was evaluated using a fully automatic contact angle analyzer (device name: DSA30, manufactured by KRÜSS GmbH). The value after 1 second from the contact of the bubbles was used as the air-bubble contact angle.

### 4-1-2. Water contact angle

The water contact angle of the obtained coating layer surface (in the case of cell culture vessel 1-10, the surface of the resin sheet on the side where cells are to be disposed) was measured according to JIS R 3257:1999. Under constant temperature and humidity conditions of 25 ± 5°C and 50 ± 10% RH, a water droplet of 1.8 µL of distilled water was dropped onto the above-described surface, and the angle of the contact interface between the surface and the water droplet within 1 minute from the dropping was measured with a contact angle meter (A-XE type, manufactured by Kyowa Interface Science Co., Ltd.). The measurement position was set to the center of the well, and the average of the values obtained by performing the measurement at three wells was used as the water contact angle of the cell culture vessel. The water contact angle was measured immediately after the formation of the coating layer.

### 4-1-3. Coating uniformity of coating material

A collagen solution of 0.3 mg/ml was applied to the surface of the coating layer to form a collagen layer. The formed collagen layer was colored red by a picrosirius red staining kit. The coloring state was visually confirmed, and the coating uniformity was evaluated according to the following criteria.

⊚: The coloring states of the peripheral portion and the central portion were the same in any of the holes of the cell culture vessel, and the coloring was uniform.

○: There were cases where the peripheral portion was colored darker than the central portion depending on the holes of the cell culture vessel.

×: There were holes that were not colored.

### 4-1-4. Oxygen transmission rate and oxygen permeability coefficient

The oxygen transmission rate and the oxygen permeability coefficient of the resin sheet after the formation of the coating layer were measured by a differential pressure type gas permeability measuring device (manufactured by Toyo Seiki Seisaku-sho, Ltd., BT-3MT-C3) in an environment of a temperature of 23°C and a humidity of 0% RH. The measurement section diameter was set to 70 mm (permeation area: 38.47 cm²). Since it was expected that the oxygen permeability coefficient would be large, an aluminum mask was applied to the sample in advance, and the actual permeation area was set to 5.0 cm².

### 4-1-5. Cell adhesiveness

The following materials were prepared.
- Laminin 1: Laminin derived from mouse (laminin full length, manufactured by Corning Incorporated)
- Laminin 2: iMatrix-511 (laminin α5β1γ1 fragment, manufactured by Matricesome)

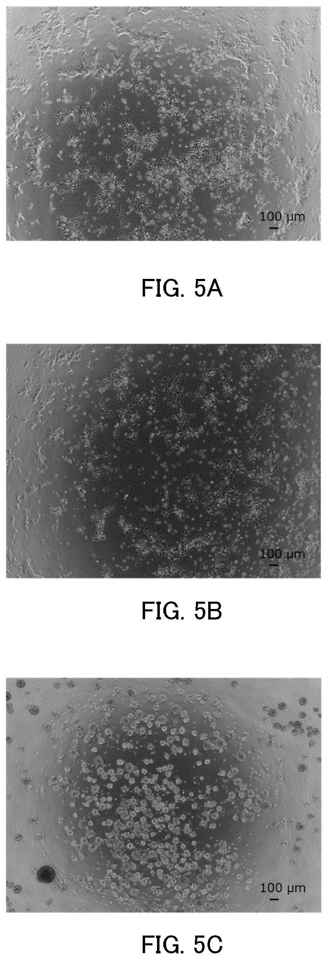

First, 0.2 mL of a 1.5 mg/mL laminin 1 aqueous solution was added to the surface of the coating layer, and the coating layer was allowed to stand at 37°C for 60 minutes and then washed with Dulbecco's PBS (-). Next, a culture solution (0.5 mL) containing human iPS cells was seeded on each culture surface of the cell culture vessel with a micropipette, a polystyrene lid was placed thereon, and the cell culture vessel was placed in an incubator to start culturing at 37°C and 5% CO₂. After 4 days, the cell culture vessel was taken out from the incubator, each culture surface was observed with a microscope, the state in which the cells adhered and spread was observed, and the adhesiveness of the cells was evaluated according to the following criteria. Microscopic photographs of cells after culturing the cells in a cell culture vessel for 4 days are observed with a phase-contrast microscope and are shown in FIGS. 5A to 5C. FIG. 5A is a microscopic photograph of a cell culture vessel with an evaluation of ⊚, FIG. 5B is a microscopic photograph of a cell culture vessel with an evaluation of ○, and FIG. 5C is a microscopic photograph of a cell culture vessel with an evaluation of ×.

⊚: The cells were sufficiently adhered and spread, and the state thereof was uniform within and between wells.

○: Although there were some uneven areas within and between wells, cells adhered and spread.

△: There were many uneven areas within and between wells.

×: The cells did not adhere in most regions of the entire well and formed a cell mass.

### 4-2. Evaluation of Experiment 2

### 4-2-1. Air-bubble contact angle in Water

The air-bubble contact angle in water of the coating layer surface (in the case of cell culture vessel 2-10, the surface of the resin sheet on the side where cells are to be disposed) and on the base material surface before and after terminal sterilization was measured in the same manner as in Experiment 1.

### 4-2-2. Water contact angle

The water contact angles of the base material and the coating layer surface (in the case of cell culture vessel 2-10, the surface of the resin sheet on the side where cells are to be disposed) were measured in the same manner as in Experiment 1. The water contact angle of the base material and the coating layer was measured immediately after the coating layer was formed.

### 4-2-3. Oxygen transmission rate and oxygen permeability coefficient

The oxygen transmission rate and the oxygen permeability coefficient of the resin sheet after terminal sterilization were measured in the same manner as in Experiment 1.

### 4-2-4. Cell adhesiveness

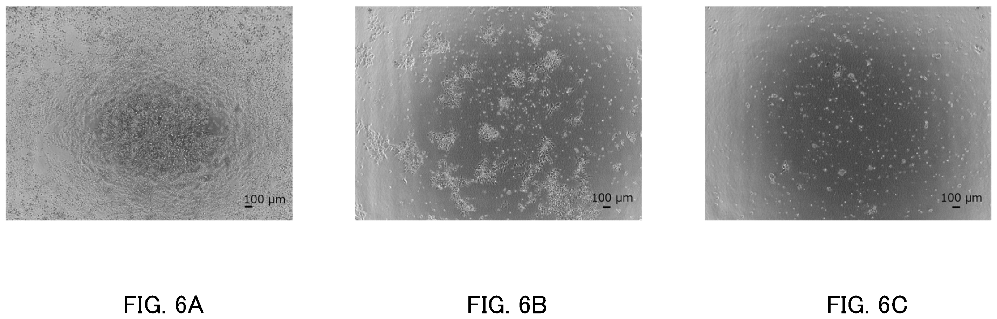

The measurement was performed using either laminin 1 or laminin 2 in the same manner as in Experiment 1. Microscopic photographs of the cells after culturing for 4 days in the cell culture vessel, observed with a phase-contrast microscope, are shown in FIGS. 6A to 6C. FIG. 6A is a microscopic photograph of a cell culture vessel with an evaluation of ⊚, FIG. 6B is a microscopic photograph of a cell culture vessel with an evaluation of ○, and FIG. 6C is a microscopic photograph of a cell culture vessel with an evaluation of ×.

Tables 1 and 2 show the materials and evaluation results of the cell culture vessels in Experiment 1, and Tables 3 to 5 show the materials and evaluation results of the cell culture vessels in Experiment 2. In Tables 1 to 5, the unit of the oxygen permeability coefficient is cm³ × mm/(m² × 24 h × atm), and the unit of the oxygen transmission rate is cm³/(m² × 24 h × atm).

**[Table 1]**

| Note | | Example | Example | Example | Example |
|---|---|---|---|---|---|
| Cell culture vessel No. | | 1-1 | 1-2 | 1-3 | 1-4 |
| Frame | Material | PS | PS | PS | PS |
| | Glass transition point [°C] | 94 | 94 | 94 | 94 |
| Base material (bottom surface) | Material | TPX | TPX | TPX | TPX |
| | Glass transition point [°C] | 40 | 40 | 40 | 40 |
| | Thickness [µm] | 50 | 50 | 50 | 50 |
| | Oxygen permeability coefficient [cm³ × mm/(m² × 24 h × atm)] | 1912 | 1912 | 1912 | 1912 |
| | Oxygen transmission rate [cm³/(m² × 24 h × atm)] | 38240 | 38240 | 38240 | 38240 |
| Coating material | Coating material | 1-1 | 1-2 | 1-3 | 1-4 |
| | Material | PHEMA | PHEMA | PHEMA | PHEMA |
| | Number-average molecular weight | 96100 | 96100 | 96100 | 96100 |
| | Solvent Water/ethanol (volume ratio) | 50/50 | 50/50 | 50/50 | 50/50 |
| | Concentration [mg/mL] | 0.005 | 0.01 | 0.05 | 0.1 |
| Coating layer | Area of well bottom [cm²] | 1.9 | 1.9 | 1.9 | 1.9 |
| | Coating amount [µl] | 200 | 200 | 200 | 200 |
| | Coating weight [µg/cm²] | 1 | 2 | 10 | 20 |
| | Air-bubble contact angle in water [°] | 48 | 43 | 35 | 32 |
| | Water contact angle (initial) [°] | 51 | 45 | 44 | 46 |
| | Coating uniformity | ⊚ | ⊚ | ⊚ | ⊚ |
| | Oxygen permeability coefficient [cm³ × mm/(m² × 24 h × atm)] | 1400 | 1400 | 1500 | - |
| | Oxygen transmission rate [cm³/(m2 × 24 h × atm)] | 28200 | 29100 | 30200 | - |
| Culture substrate layer | Material | Laminin | Laminin | Laminin | Laminin |
| | Cell adhesiveness | ○ | ○ | ⊚ | ⊚ |

**[Table 2]**

| Note | | Example | Example | Example |
|---|---|---|---|---|
| Cell culture vessel No. | | 1-5 | 1-6 | 1-7 |
| Frame | Material | PS | PS | PS |
| | Glass transition point [°C] | 94 | 94 | 94 |
| Base material (bottom surface) | Material | TPX | TPX | TPX |
| | Glass transition point [°C] | 40 | 40 | 40 |
| | Thickness [µm] | 50 | 50 | 50 |
| | Oxygen permeability coefficient [cm³ × mm/(m² × 24 h × atm)] | 1912 | 1912 | 1912 |
| | Oxygen transmission rate [cm³/(m² × 24 h × atm)] | 38240 | 38240 | 38240 |
| Coating material | Coating material | 1-5 | 1-6 | 1-7 |
| | Material | Gelatin | Gelatin | Gelatin |
| | Number-average molecular weight | - | - | - |
| | Solvent Water/ethanol (volume ratio) | 100/0 | 100/0 | 100/0 |
| | Concentration [mg/mL] | 0.001 | 0.01 | 0.3 |
| Coating layer | Area of well bottom [cm²] | 1.9 | 1.9 | 1.9 |
| | Coating amount [µl] | 200 | 200 | 200 |
| | Coating weight [µg/cm²] | 0.2 | 2 | 60 |
| | Air-bubble contact angle in water [°] | 49 | 48 | 36 |
| | Water contact angle (initial) [°] | 88 | 80 | 51 |
| | Coating uniformity | ○ | ○ | ○ |
| | Oxygen permeability coefficient [cm³ × mm/(m² × 24 h × atm)] | 1400 | - | 200 |
| | Oxygen transmission rate [cm³/(m² × 24 h × atm)] | 27500 | - | 3780 |
| Culture substrate layer | Material | Laminin | Laminin | Laminin |
| | Cell adhesiveness | ○ | ○ | ⊚ |

**[Table 3]**

| Note | | Example | Example | Example |
|---|---|---|---|---|
| Cell culture vessel No. | | 2-1 | 2-2 | 2-3 |
| Frame | Material | PS | PS | PS |
| | Glass transition point [°C] | 94 | 94 | 94 |
| Base material | Material | 4M1P | 4M1P | 4M1P |
| | Air-bubble contact angle (after sterilization) [°] | 106 | 106 | 106 |
| | Glass transition point [°C] | 40 | 40 | 40 |
| | Thickness [µm] | 50 | 50 | 50 |
| | Oxygen permeability coefficient | 1912 | 1912 | 1912 |
| | Oxygen transmission rate | 38240 | 38240 | 38240 |
| Coating material | Coating material | 2-1 | 2-2 | 2-3 |
| | Material | PMEA 1 | PMEA 2 | PMEA 1 |
| | Number-average molecular weight | 74000 | 305000 | 74000 |
| | Solvent Water/ethanol (volume ratio) | 50/50 | 50/50 | 50/50 |
| | Concentration [µg/mL] | 30 | 30 | 30 |
| Coating layer | Area of well bottom [cm²] | 1.9 | 1.9 | 1.9 |
| | Coating amount [µL] | 200 | 200 | 200 |
| | Air-bubble contact angle (initial) [°] | 53 | 51 | 53 |
| | Air-bubble contact angle (after sterilization) [°] | 57 | 47 | 57 |
| | Water contact angle (initial) [°] | 52 | 89 | 52 |
| | Difference of air-bubble contact angle with respect to base material (after sterilization) [°] | 49 | 59 | 49 |
| Oxygen permeation section | Oxygen permeability coefficient | 1400 | 1400 | 1400 |
| | Oxygen transmission rate | 27800 | 28100 | 27800 |
| Culture substrate layer | Material | Laminin 2 | Laminin 2 | Laminin 1 |
| Sterilization | Radiation | γ-ray | γ-ray | γ-ray |
| | Absorbed dose [kGy] | 12 | 12 | 12 |
| Evaluation | Cell adhesiveness | ⊚ | ⊚ | ○ |

**[Table 4]**

| Note | | Example | Example | Example | Example |
|---|---|---|---|---|---|
| Cell culture vessel No. | | 2-4 | 2-5 | 2-6 | 2-7 |
| Frame | Material | PS | PS | PS | PS |
| | Glass transition point [°C] | 94 | 94 | 94 | 94 |
| Base material | Material | 4M1P | 4M1P | 4M1P | 4M1P |
| | Air-bubble contact angle (after sterilization) [°] | 106 | 106 | 106 | 106 |
| | Glass transition point [°C] | 40 | 40 | 40 | 40 |
| | Thickness [µm] | 50 | 50 | 50 | 50 |
| | Oxygen permeability coefficient | 1912 | 1912 | 1912 | 1912 |
| | Oxygen transmission rate | 38240 | 38240 | 38240 | 38240 |
| Coating material | Coating material | 2-4 | 1-5 | 2-6 | 2-7 |
| | Material | PHEMA | Gelatin | PMEA 1 /PHEMA | PMEA 1 /PHEMA |
| | Number-average molecular weight | 96100 | - | - | - |
| | Solvent Water/ethanol (volume ratio) | 50/50 | 50/50 | 50/50 | 50/50 |
| | Concentration [µg/mL] | 4 | 5 | 30 | 30 |
| Coating layer | Area of well bottom [cm²] | 1.9 | 1.9 | 1.9 | 1.9 |
| | Coating amount [µL] | 200 | 200 | 200 | 200 |
| | Air-bubble contact angle (initial) [°] | 45 | 45 | 62 | 61 |
| | Air-bubble contact angle (after sterilization) [°] | 44 | 59 | 59 | 60 |
| | Water contact angle (initial) [°] | 48 | 76 | 74 | 72 |
| | Difference of air-bubble contact angle with respect to base material (after sterilization) [°] | 62 | 47 | 47 | 46 |
| Oxygen permeation section | Oxygen permeability coefficient | 1400 | 1400 | 1400 | 1400 |
| | Oxygen transmission rate | 28000 | 28300 | 27800 | 27800 |
| Culture substrate layer | Material | Laminin 1 | Laminin 1 | Laminin 2 | Laminin 2 |
| Sterilization | Radiation | γ-ray | γ-ray | γ-ray | γ-ray |
| | Absorbed dose [kGy] | 12 | 12 | 12 | 12 |
| Evaluation | Cell adhesiveness | ○ | ⊚ | ⊚ | ⊚ |

**[Table 5]**

| Note | | Comparative example | Comparative example | Comparative example |
|---|---|---|---|---|
| Cell culture vessel No. | | 2-8 | 2-9 | 2-10 |
| Frame | Material | PS | PS | PS |
| | Glass transition point [°C] | 94 | 94 | 94 |
| Base material (Bottom surface) | Material | 4M1P | 4M1P | 4M1P |
| | Air-bubble contact angle (after sterilization) [°] | 106 | 106 | 106 |
| | Glass transition point [°C] | 40 | 40 | 40 |
| | Thickness [µm] | 50 | 50 | 50 |
| | Oxygen permeability coefficient | 1912 | 1912 | 1912 |
| | Oxygen transmission rate | 38240 | 38240 | 38240 |
| Coating material | Coating material | 1-8 | 1-9 | None |
| | Material | Collagen | Collagen | None |
| | Number-average molecular weight | - | - | - |
| | Solvent Water/ethanol (volume ratio) | 100/0 | 100/0 | - |
| | Concentration [µg/mL] | 0.05 | 0.3 | - |
| Coating layer | Area of well bottom [cm²] | 1.9 | 1.9 | 1.9 |
| | Coating amount [µL] | 200 | 200 | - |
| | Air-bubble contact angle (initial) [°] | 10 | 60 | 106 |
| | Air-bubble contact angle (after sterilization) [°] | 28 | 28 | 106 |
| | Water contact angle (initial) [°] | 56 | 55 | 101 |
| | Difference of air-bubble contact angle with respect to base material (after sterilization) [°] | 78 | 78 | 5 |
| Oxygen permeation section | Oxygen permeability coefficient | 1200 | 100 | 1912 |
| | Oxygen transmission rate | 22700 | 1650 | 38240 |
| Culture substrate layer | Material | Laminin 1 | Laminin 1 | Laminin 1 |
| Sterilization | Radiation | γ-ray | γ-ray | γ-ray |
| | Absorbed dose [kGy] | 12 | 12 | 12 |
| Evaluation | Cell adhesiveness | × | × | × |

As is clear from Tables 1 to 5, a cell culture vessel that is made of a resin and includes a oxygen permeation section having an oxygen permeability coefficient of 200 cm³ × mm/(m² × 24 h × atm) or more and 2,400 cm³ × mm/(m² × 24 h × atm) or less and a coating layer having an air-bubble contact angle in water of 30° or more and 100° or less has high cell adhesiveness.

This application is entitled to and claims the benefits of Japanese Patent Application No. 2024-002531 filed on January 11, 2024 and Japanese Patent Application No. 2024-171160 filed on September 30, 2024, the disclosures of which each including the specification, claims, and drawings are incorporated herein by reference in their entirety.

### Industrial Applicability

The cell culture vessel according to the present invention can be applied to the culture of various cells.

### Reference Signs List

100 Cell culture vessel
200 Base material
210 Accommodating section
220 Frame
222 Frame section
224 Well
230 Resin sheet
240 Pressure-sensitive adhesive layer
300 Coating layer
500 Cell culture vessel
520 Frame
522 Frame section
528 Bottom plate section
524a, 524b Chamber
524c Flow path
530 Resin sheet
540 Cell body
542 Axon
544 Neural tissue

## Claims

1. A cell culture vessel, comprising:
a base material including an oxygen permeation section; and
a coating layer disposed on a surface of the base material, the surface being on a side on which cells are to be disposed, wherein
the oxygen permeation section is made of resin and has an oxygen permeability coefficient of 200 cm³ × mm/(m² × 24 h × atm) or more and 2400 cm³ × mm/(m² × 24 h × atm) or less, and
the coating layer has an air-bubble contact angle in water of 30° or more and 100° or less.

2. The cell culture vessel according to claim 1, wherein a difference between the air-bubble contact angle in water of the coating layer and an air-bubble contact angle in water of the oxygen permeation section is 35° or more and 75° or less.

3. The cell culture vessel according to claim 1, wherein the coating layer has a water contact angle of 40° or more and 90° or less.

4. The cell culture vessel according to claim 1, wherein the coating layer contains a polyalkoxyalkyl (meth)acrylate and/or a polyhydroxyalkyl (meth)acrylate.

5. The cell culture vessel according to claim 4, wherein the polyalkoxyalkyl (meth)acrylate contains at least one compound selected from the group consisting of polymethoxymethyl acrylate, poly(2-methoxyethyl acrylate), polymethoxypropyl acrylate, polymethoxybutyl acrylate, polyethoxymethyl acrylate, polyethoxyethyl acrylate, polyethoxypropyl acrylate, polyethoxybutyl acrylate, polypropoxymethyl acrylate, polypropoxyethyl acrylate, polypropoxypropyl acrylate, polypropoxybutyl acrylate, polybutoxymethyl acrylate, polybutoxyethyl acrylate, polybutoxypropyl acrylate, polybutoxybutyl acrylate, polymethoxymethyl methacrylate, polymethoxyethyl methacrylate, polymethoxypropyl methacrylate, polymethoxybutyl methacrylate, polyethoxymethyl methacrylate, polyethoxyethyl methacrylate, polyethoxypropyl methacrylate, polyethoxybutyl methacrylate, polypropoxymethyl methacrylate, polypropoxyethyl methacrylate, polypropoxypropyl methacrylate, polypropoxybutyl methacrylate, polybutoxymethyl methacrylate, polybutoxyethyl methacrylate, polybutoxypropyl methacrylate, and polybutoxybutyl methacrylate.

6. The cell culture vessel according to claim 4, wherein the polyalkoxyalkyl (meth)acrylate and/or the polyhydroxyalkyl (meth)acrylate has a number-average molecular weight Mn of 10,000 or more and 500,000 or less.

7. The cell culture vessel according to claim 1, wherein the coating layer contains gelatin.

8. The cell culture vessel according to claim 1, wherein the coating layer has a coating weight of 0.1 µg/cm² or more and 100 µg/cm² or less.

9. The cell culture vessel according to claim 1, wherein the base material contains at least one resin selected from the group consisting of polystyrene, polyethylene, polypropylene, 4-methyl-1-pentene-based (co)polymers, and cyclic olefin-based (co)polymers.

10. The cell culture vessel according to claim 1, wherein the oxygen permeation section has an air-bubble contact angle in water of 100° or more.

11. The cell culture vessel according to claim 1, wherein the oxygen permeation section contains at least one resin selected from the group consisting of polyethylene, polypropylene, polyethylene terephthalate, and 4-methyl-1-pentene-based (co)polymers.

12. The cell culture vessel according to claim 1, wherein the base material includes a frame and a resin sheet, the frame including a through-hole, the resin sheet being disposed on an upper surface or a bottom surface of the through-hole.

13. The cell culture vessel according to claim 12, wherein the oxygen permeation section is formed on the resin sheet.

14. The cell culture vessel according to claim 12, wherein the resin sheet has a thickness of 10 µm or more and 500 µm or less.

15. The cell culture vessel according to claim 12, wherein the resin sheet is formed of a thermoplastic resin of a type different from that of the base material.

16. The cell culture vessel according to claim 1, wherein a sterility assurance level (SAL) of a medical device, as measured in accordance with BS EN 556-1:2001, is 10⁻² or less.

17. A method for manufacturing a cell culture vessel, the method comprising:
preparing a base material including an oxygen permeation section; and
disposing a coating layer by applying a coating material to a surface of the base material and drying the applied coating material, the surface being on a side on which cells are to be cultured, wherein
the oxygen permeation section is made of a resin and has an oxygen permeability coefficient of 200 cm³ × mm/(m² × 24 h × atm) or more and 2,400 cm³ × mm/(m² × 24 h × atm) or less, and
the disposed coating layer has an air-bubble contact angle in water of 30° or more and 100° or less.

18. The method according to claim 17, further comprising:
packaging the cell culture vessel after the coating layer is disposed; and
subjecting the packaged cell culture vessel to sterilization treatment.

19. The method according to claim 17, wherein the coating layer contains a polyalkoxyalkyl (meth)acrylate and/or a polyhydroxyalkyl (meth)acrylate.

20. The method according to claim 17, wherein the coating material contains gelatin and a solvent.

21. A method for culturing a cell, the method comprising:
preparing the cell culture vessel according to any one of claims 1 to 16;
forming a culture substrate layer on a surface of the coating layer, the culture substrate layer containing a natural polymer selected from the group consisting of collagen, laminin, laminin fragments, fibronectin, vitronectin, elastin, tenascin, entactin, fibrillin, and proteoglycans; and
culturing the cell in the cell culture vessel.
